# EUROPEAN PATENT SPECIFICATION

(11) **EP 3 964 399 B1**
(45) Date of publication and mention of the grant of the patent: **21.02.2024**
(21) Application number: 20194413.9
(22) Date of filing: 03.09.2020
(51) Int. Cl.: B60R 13/02

(54) **DEVICE FOR COUNTERACTING MOTION SICKNESS IN A VEHICLE**
VORRICHTUNG ZUR BEKÄMPFUNG VON REISEKRANKHEIT IN EINEM FAHRZEUG
DISPOSITIF POUR CONTRECARRER LA CINÉTOSE DANS UN VÉHICULE

(43) Date of publication of application: 09.03.2022
(73) Proprietor: Inalfa Roof Systems Group B.V., 5807 GW Oostrum (NL)
(72) Inventor: Vervoort, Stephan Christiaan, 5831 HB Boxmeer (NL); De Jong, Robbert Arij Jeroen, 5807 GW Oostrum (NL)
(74) Representative: De Vries & Metman

(56) References cited:
- EP-A1- 3 702 217
- WO-A1-2020/012458
- WO-A1-2020/070402
- DE-A1-102017 207 788
- DE-A1-102018 126 835
- US-A1- 2015 352 453
- US-A1- 2017 291 538
- US-A1- 2018 194 247
- US-A1- 2019 047 498
- US-A1- 2019 061 655
- US-B1- 10 589 679

## Description

The invention relates to a device for counteracting motion sickness in a vehicle.

Motion sickness is a known condition of passengers of a vehicle. A main aspect contributing to motion sickness is a deviation between visual information and experienced motion, i.e. information from the vestibular system. For example, when viewing at a display or reading a book in a vehicle, motion sickness may occur, since the display or book remains stationary relative to the visual system, while the vestibular system experiences motion. Thus, motion sickness may be suppressed by looking at the horizon during travel in a vehicle such that visual information and vestibular information correspond as much as possible.

In a known vehicle interior, an entertainment system displaying a video may be configured to move the displayed video images with the movement of the vehicle to reduce the chance of motion sickness due to the viewing of the video by generating a visual motion corresponding to the actual motion. In another known vehicle interior, the passenger seats may be actuatable to move the seat with the movement of the vehicle. Thus, both known vehicle interiors are configured to reduce the deviation between visual and vestibular information.

Another known system employs a running light along a line in the passenger compartment, e.g. along an edge of the roof, to inform a passenger on the actual movement. The system is designed to reduce motion sickness in a visually closed environment, where there is no visual information relating to the actual motion of the vehicle. For example, a future, autonomously driving vehicle may be provided with such a visually closed passenger compartment. It has been shown that such lighting information may relieve the motion sickness, but only for a minor number of the passengers and only after a period of training. The document WO2020/070402A1 discloses a roof assembly according to the preamble of claim 1.

It is an object of the present invention to provide a device for mounting in a vehicle for reducing motion sickness, wherein no training for the passengers is required.

In a first aspect, the object is achieved in a device according to claim 1. The device according to the present invention is configured to be arranged in a passenger compartment of a vehicle, wherein at least a part of the device is configured to be moveable in a predetermined limited range of movement corresponding to an acceleration of the vehicle. Further, the device is designed and configured to provide visual information to a passenger with respect to a direction of such acceleration by movement of said part corresponding to such direction.

Moving at constant velocity in a same direction is experienced as being stationary. Any acceleration is experienced by the vestibular system as movement. For example, turning of a vehicle left corresponds to an acceleration in the leftward direction. Hence, to provide visual information regarding the movement of the vehicle, wherein such visual information corresponds to the motion as experienced by the vestibular system. The movement of the visual information should correspond to the acceleration of the vehicle, wherein a direction of the movement to correspond to a direction of the actual acceleration is particular important.

The device according to the present invention is not intended to be continuously actively observed by a passenger. The device is preferably visible anywhere in a field of view of a passenger, while the passenger may be actually focussing on another object. Thus, the device according to the present invention provides a visual reference within the field of view. Of course, passengers that get motion sick easily may still focus on the device in order to increase the visual impact.

In an embodiment of the device, said part of the device is a decorative element. The part of the device that is moveable is visibly arranged in the passenger compartment and may be decorative to provide a visually attractive lining, or the like.

In an embodiment of the device, said part of the device is arranged on a roof of the passenger compartment. In order to achieve a good effect, the moveable part may be preferred to be relatively large and in a field of view of a passenger. The roof in the passenger compartment may be a good position to arrange a large moveable part within the field of view of most, if not all, passengers.

In an embodiment, said part of the device is rotatably mounted in a substantially horizontal plane such to rotate with a rotational movement of the vehicle in the substantially horizontal plane. A main cause for motion sickness in a land vehicle like a car, is turning left or right. Therefore, it may be preferred to at least ensure that such turning is reflected in a corresponding movement of the moveable part. In view of the rotating movement of the turning, the moveable part may be preferred to rotate. An exemplary embodiment may be disc-shaped part. Further, a rotating movement reduces an amount of space needed for the predetermined range of movement.

In an embodiment, said part of the device is rotatably mounted in a substantially vertical plane such to rotate with a rotational movement of the vehicle in the substantially vertical plane. Such embodiment is suited to reflect a movement of the vehicle due to acceleration or due to height differences, e.g. when driving in the mountains or when sailing. Of course, in a particular embodiment, said part may be rotatable in both the horizontal and the vertical plane. For example, the moveable part may be ball-shaped.

In an embodiment thereof, the device comprises a control unit, wherein the control unit comprises a sensor for detecting the acceleration and an actuator for moving said part of the device corresponding to the acceleration as detected. In particular, a processing unit may be provided in the control unit to receive an output of the sensor indicating an actual acceleration of the vehicle. In response to the received sensor output, the processing unit may drive an actuator operatively coupled to the moveable part of the device. The actuator is configured to move the moveable part of the device, wherein the processing unit controls the actuator such that the movement of the moveable part corresponds to the actual acceleration. Still, in a particular embodiment, the processing unit may be omitted and an output of the sensor may be suitable to drive the actuator directly.

The device comprises a support element, an elastic element and said moveable part. The support element is fixedly mounted in the passenger compartment and the elastic element is arranged between the support element and the moveable part. The moveable part is configured to move in response to acceleration-induced inertia of the moveable part such that the moveable part moves with the actual acceleration of the vehicle. The elastic element is arranged to counteract such a relative movement between the moveable part and the support element, thereby limiting the movement range. Due to the elasticity of the elastic element, the moveable part returns to its initial position and orientation, when the acceleration stops. More in general and as preferably also valid for the above mentioned embodiments, an amplitude of the movement of the moveable part corresponds to an amount of acceleration, while a direction of the movement corresponds to a direction of the acceleration.

In another embodiment, the device further comprises a sensor for detecting a condition in the passenger compartment. More and more, systems with sensors for sensing all kinds of interior conditions, e.g. temperature, passenger presence, air quality, etc. are provided in a vehicle interior. Such sensors may be preferred to be arranged in a single device. Therefore, in this embodiment of the device according to the present invention sensors including an acceleration sensor may be provided. For example, a single processing unit may be provided to receive the outputs of all the sensors, control related actuators and/or communicate with other control units in the vehicle.

In an aspect, the present invention further provides a roof assembly for a vehicle, wherein the roof assembly comprises a support frame mountable on a body frame of the vehicle to form a roof of a passenger compartment of the vehicle, wherein the roof assembly comprises the above-described device.

In a further aspect, the present invention provides a computer program product comprising instructions to cause a processing unit of at least one of the above described control units to execute the step of detecting an acceleration and the step of moving said part of the device corresponding to the acceleration as detected.

In a yet further aspect, the present invention provides a computer readable medium carrying the above-mentioned computer program product.

Further scope of applicability of the present invention will become apparent from the detailed description given hereinafter. However, it should be understood that the detailed description and specific examples, while indicating embodiments of the invention, are given by way of illustration only, since various changes and modifications within the scope of the invention will become apparent to those skilled in the art from this detailed description with reference to the appended schematical drawings, in which:
- Fig. 1A: shows a perspective view of a vehicle roof with an open roof assembly;
- Fig. 1B: shows an exploded view of the open roof assembly of Fig. 1A;
- Fig. 2A: shows a perspective view on a first embodiment of a device according to the present invention;
- Fig. 2B: shows a top view of a vehicle comprising the device according to Fig. 2A;
- Figs. 3A - 3B: illustrate exemplary details of the first embodiment according to Fig. 2A.
- Fig. 4: shows a diagram illustrating a method of operating a device according to the present invention;

The present invention will now be described with reference to the accompanying drawings, wherein the same reference numerals have been used to identify the same or similar elements throughout the several views.

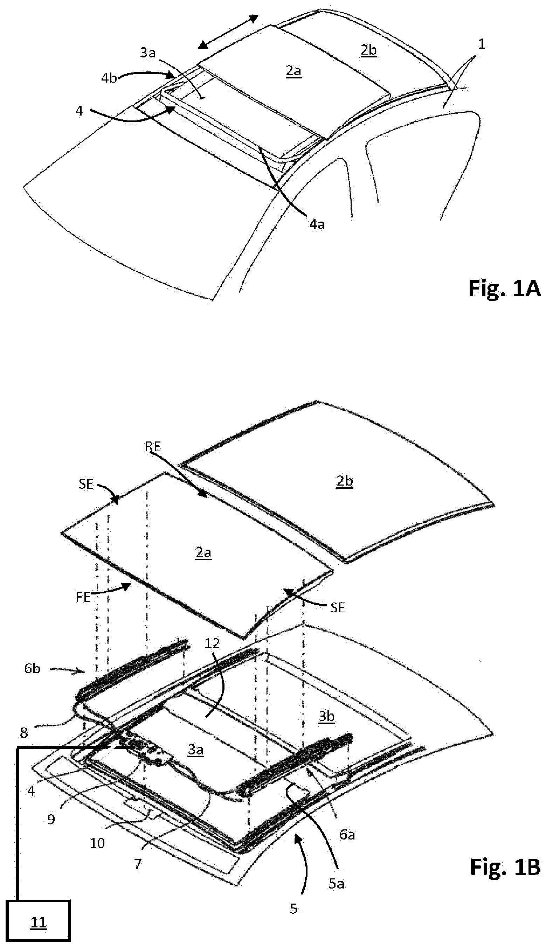

Fig. 1A illustrates a vehicle roof 1 having an open roof assembly arranged therein. The open roof assembly comprises a moveable panel 2a and a fixed panel 2b. The moveable panel 2a is also referred to as a closure member, since the moveable panel 2a is moveable over a first roof opening 3a such to enable to open and to close the first roof opening 3a. A wind deflector 4 is arranged at a front side of the first roof opening 3a.

In the illustrated embodiment, the moveable panel 2a may be in a closed position, which is a position wherein the moveable panel 2a is arranged over and closes the first roof opening 3a and thus usually is arranged in a plane of the vehicle roof 1. Further, the moveable panel 2a may be in a tilted position, which is a position wherein a rear end RE of the moveable panel 2a is raised as compared to the closed position, while a front end FE of the moveable panel 2a is still in the closed position. Further, the moveable panel 2a may be in an open position, which is a position wherein the moveable panel 2a is slid open and the first roof opening 3a is partly or completely exposed.

It is noted that the illustrated vehicle roof 1 corresponds to a passenger car. The present invention is however not limited to passenger cars. Any other kind of vehicles that may be provided with a moveable panel are contemplated as well.

Fig. 1B illustrates the same vehicle roof as shown in Fig. 1A having panels 2a and 2b. In particular, while Fig. 1A shows the open roof assembly in the open position, Fig. 1B is an exploded view of the open roof assembly in a closed position. Further, in this exploded view of Fig. 1B, it is shown that there is a second roof opening 3b. The first and second roof openings 3a, 3b are provided in a frame 5 of the open roof assembly, having a middle beam 12 of the frame 5 between the openings 3a, 3b. An edge 5a of the frame 5 defines the first roof opening 3a.

The second roof opening 3b is arranged under the fixed panel 2b such that light may enter a vehicle interior passenger compartment through the fixed panel 2b, presuming that the fixed panel 2b is a glass panel or a similarly transparent panel, for example made of a plastic material or any other suitable material. The second roof opening 3b with a transparent or translucent fixed panel 2b is optional and may be omitted in another embodiment of the open roof assembly.

The wind deflector 4 is commonly a flexible material, e.g. a woven or non-woven cloth having through holes arranged therein or a web or net. The flexible material is supported by a support structure 4a, e.g. a bar-like or tube-like structure, which structure is hingedly coupled, directly or indirectly, to the frame 5 at a hinge 4b.

The wind deflector 4 is arranged in front of the first roof opening 3a and adapts air flow when the moveable panel 2a is in the open position. In its raised position, the wind deflector 4 reduces inconvenient noise due to air flow during driving. When the moveable panel 2a is in the closed position or in the tilted position, the wind deflector 4 is held down below the front end FE of the moveable panel 2a.

Usually, the wind deflector 4 is raised by a spring force when the moveable panel 2a slides to an open position and the wind deflector 4 is pushed down by the moveable panel 2a when the moveable panel 2a slides back into its closed position. In Fig. 1A, the moveable panel 2a is shown in an open position and the wind deflector 4 is shown in a raised position. In Fig. 1B, the moveable panel 2a is shown in a closed position and the wind deflector 4 is correspondingly shown in a position in which it is held down.

Fig. 1B further illustrates a drive assembly having a first guide assembly 6a, a second guide assembly 6b, a first drive cable 7 and a second drive cable 8. The first and second guide assemblies 6a, 6b are arranged on respective side ends SE of the moveable panel 2a and may each comprise a guide and a mechanism. The guide is coupled to the frame 5, while the mechanism comprises moveable parts and is slideably moveable in the guide. The first and the second drive cables 7, 8 are provided between the mechanisms of the respective guide assemblies 6a, 6b and a electric motor 9.

The drive cables 7, 8 couple the electric motor 9 to the mechanisms of the respective guide assemblies 6a, 6b such that upon operating the electric motor 9, the mechanisms start to move. In particular, a core of the drive cable 7, 8 is moved by the electric motor 9 such to push or pull on the mechanisms of the respective guides 6a, 6b. Such a drive assembly is well known in the art and is therefore not further elucidated herein. Still, any other suitable drive assembly may be employed as well without departing from the scope of the present invention. Moreover, in a particular embodiment, an electric motor may be operatively arranged between the respective guides and the respective mechanisms of the guide assemblies 6a, 6b and, in such embodiment, a drive assembly may be omitted completely.

In the illustrated embodiment, the guide assemblies 6a, 6b may start movement with raising the rear end RE of the moveable panel 2a, thereby bringing the moveable panel 2a in the tilted position. Then, from the tilted position, the guide assemblies 6a, 6b may start to slide to bring the moveable panel 2a in the open position. The present invention is however not limited to such embodiment. For example, in another embodiment, the moveable panel 2a may be moveable to a tilted position by raising the rear end RE, while an open position is reached by first lowering the rear end RE and then sliding the moveable panel 2a under the fixed panel 2b or any other structure or element provided behind the rear end RE of the moveable panel 2a. In further exemplary embodiments, the moveable panel 2a may be merely moveable between a closed position and a tilted position or between a closed position and an open position.

In the illustrated embodiment, the electric motor 9 is mounted near or below the front end FE of the moveable panel 2a at a recess 10. In another embodiment, the electric motor 9 may be positioned at any other suitable position or location. For example, the electric motor 9 may be arranged near or below the rear end RE of the moveable panel 2a or below the fixed panel 2b.

A control module 11 is schematically illustrated and is operatively coupled to the electric motor 9. The control module 11 may be any kind of processing module, either a software controlled processing module or a dedicated processing module, like an ASIC, which are both well known to those skilled in the art. The control module 11 may be a stand-alone control module or it may be operatively connected to another control module, like a multipurpose, generic vehicle control module. In yet another embodiment, the control module 11 may be embedded in or be part of such a generic vehicle control module. Essentially, the control module 11 may be embodied by any control module suitable for, capable of and configured for performing operation of the electric motor 9 and thus the moveable roof assembly.

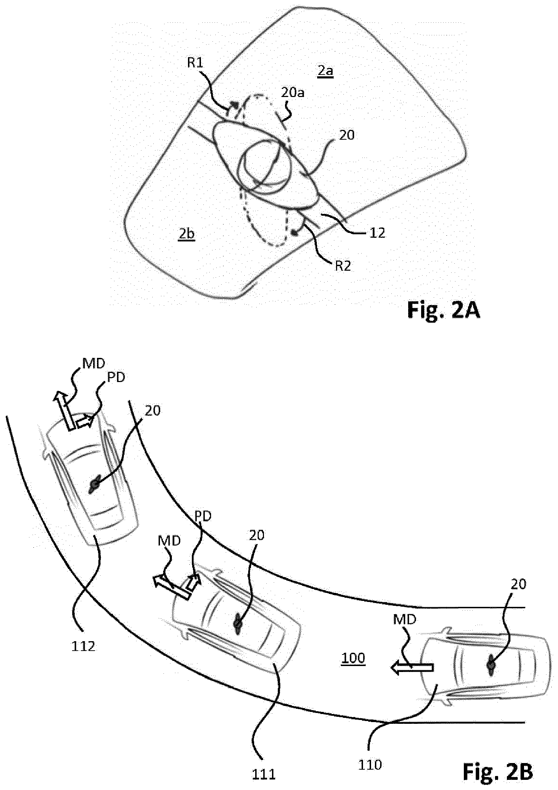

Fig. 2A shows the open roof assembly of Figs. 1A and 1B from an interior side of the vehicle. The open roof assembly is provided with a device 20, which is arranged on the middle beam 12. In this embodiment, the device 20 is oval shaped and rotatably mounted on the middle beam 12 such that the device 20 may rotate in a clockwise direction as indicated by arrows R1, R2 to a position 20a. Similarly, the device 20 may rotate in an anticlockwise direction. The rotation and resulting position corresponds to a driving direction of the vehicle as illustrated in Fig. 2B.

When driving in a straight direction on a road 100 as illustrated by a first vehicle 110, the oval device 20 is oriented parallel to the middle beam. When the vehicle is turning like second and third vehicles 111, 112, respectively, the device 20 is rotated relative to the middle beam, indicating to any occupant of the vehicle that the vehicle is turning.

The amount of rotation of the device 20 corresponds to a radius of the turning of the vehicle. For example, the rotation angle of the device 20 relative to the middle beam in the second vehicle 111 is such that the device 20 - in Fig. 2B - might appear to be parallel to the device 20 of the first vehicle 110, but this is purely coincidental. As apparent from a comparison between the second and third vehicles 111 and 112, the rotation angle of the device 20 relative to the middle beam remains constant with a constant radius of the turning of the vehicle.

More in detail, the rotation angle of the device 20 is determined by the acceleration of the vehicle and in particular an acceleration in a perpendicular direction PD perpendicular to a main, straight driving direction MD. The first vehicle 110 drives in the main, straight direction MD and may or may not be accelerating or decelerating. The second and third vehicles 111, 112 are turning and thus have an acceleration in the perpendicular direction PD. Thus, a total acceleration is directed in a direction deviating from the main driving direction MD and the device 20 adapts its rotation angle to the direction of the total acceleration, for example. In another embodiment, the device 20 may be designed and configured to reflect only the acceleration in the perpendicular direction PD, although such indicated direction may not correspond to the natural feeling of the occupants.

In general, with the device 20, occupants in the vehicle are provided with a natural reference to the actual driving direction of the vehicle in the passenger compartment of the vehicle. Thereby, visual information regarding the driving direction and the vestibular information correspond and motion sickness may be suppressed.

The illustrated embodiment of a rotating device 20 at a middle beam of an open roof assembly is particularly suitable for suppressing motion sickness of passengers on a backseat of a vehicle as front passengers do not have the device 20 in their field of view when sitting in front looking out the front windscreen. Still, such front passengers usually have sufficient natural references from the outside scenery. In future, autonomously driving vehicles may have limited views to the scenery or front passengers may be looking rearward, while driving, in which case the illustrated embodiment of the device 20 may be sufficient to suppress motion sickness also for the front passengers.

Of course, in another embodiment, the device may be shaped differently. It may be larger, occupying a larger area of the roof of the interior passenger compartment or there may be multiple devices, either arranged on the interior roof or at other locations in the passenger compartment in eyesight of the passengers.

Further, the device 20 may be configured to respond to other orientations of the vehicle e.g. an orientation in a vertical plane. For example, an orientation of the device may correspond to a tilt angle of the vehicle. For example, when the vehicle drives up or down a hill, the device may remain in a horizontal orientation, thereby showing an inclination angle of the vehicle relative to the horizon. Multiple angles and rotations may be shown by a single device or multiple devices may be provided in a vehicle, each showing at least one orientation or driving direction of the vehicle.

In the illustrated embodiment, the device 20 is arranged on a middle beam 12 of an open roof assembly. In another embodiment, the device 20 may be arranged on any other structure in a passenger compartment of a vehicle. It may be arranged on an interior surface of a vehicle roof with or without a transparent panel arranged in the roof or it may be arranged on a dashboard or any other surface or structure. When arranged on the roof, it may be arranged in a centre part of the roof or at any other position on the interior surface. In an exemplary, particular embodiment, the device 20 may be arranged such that it is flush with the interior surface of the roof, wherein a decorative look of a visible surface of the device 20 provides the visual information to a passenger. Of course, such a decorative look may as well be provided on a surface of the device 20 in any other embodiment, such as the embodiment of Fig. 2A.

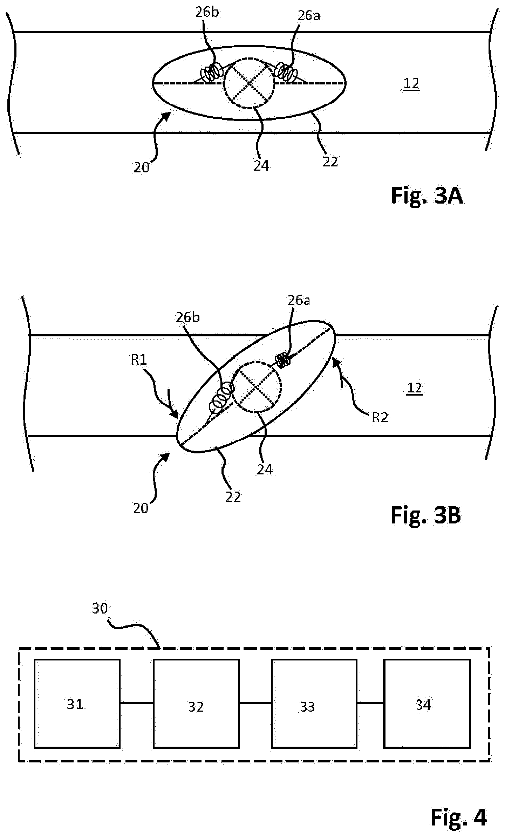

Fig. 3A illustrates a particular exemplary embodiment of the device 20 as shown in Figs. 2A and 2B. The device 20 comprises a base 24 and a cover 22. The base 24 is fixedly arranged on the middle beam 12 and forms a support element for the cover 22. The cover 22 forms a moveable part rotatably mounted on the base 24.

A set of springs 26a, 26b is provided as an elastic element. The set of springs 26a, 26b is mechanically coupled between the base 24 and the cover 22. The set of springs 26a, 26b are not essential for the functioning of the device 20. Advantageously, the set of springs 26a, 26b may be provided to compensate for friction between the cover 22 and the base 24. The set of springs 26a, 26b are arranged and biased such that in absence of any external forces, the cover 22 is in a starting position, which corresponds to the position as shown in Fig. 3A.

The cover 22 may be designed such that a centre of gravity of the cover 22 is aligned with a centre of rotation in the main driving direction, thereby ensuring that the cover 22 is in the starting position, when the vehicle is driving in the main driving direction, i.e. when driving straight forward. If the centre of gravity does not coincide with the centre of rotation, however, as soon as the vehicle starts to turn during driving, a torque is generated and the cover 22 will rotate against a spring force of the set of springs 26a, 26b.

As illustrated in Fig. 3B, a first spring 26a may be compressed due to the rotation, while a second spring 26b may be extended. Preferably, the spring force of the set of springs 26a, 26b is relatively low to minimize a counterforce to the inertia force generated by the acceleration. On the other hand, the spring force is preferred to be larger than a friction in the device 20 such that the cover 22 rotates to the starting position as soon as the inertia force is removed.

A person skilled in the art readily recognizes that many other technical means to achieve and provide such return to the starting position are well known and may function equally well. Such technical means include different kinds of springs or other elastic elements. As well, other technical means may be selected instead of an elastic element. For example, the cover 22 may rotate along a helical guide track on the base 24 upward against gravity such that gravity will pull the cover 22 back to the starting position, if and as soon as the inertia force is removed.

Fig. 4 illustrates another embodiment of a device 30, which does not rely on induced mechanical forces as the above-described embodiment 20. The device 30 is based on electronic control. The illustrated device 30 comprises a sensor 31, a control unit 32, an actuator 33 and a moveable element 34, which is visible to passengers.

In an embodiment, the sensor 31 may be an acceleration sensor detecting the actual acceleration. In another embodiment, the sensor 31 may be a sensor detecting an orientation of one or more wheels of the vehicle or an orientation of the steering wheel. Any other sensor or a combination of sensors may be used for detecting an orientation or acceleration during driving of the vehicle for accordingly adapting an orientation of the moveable element 34.

The control unit 32 receives a sensor output and determines based on such output and possibly other data received from another system or device a desired orientation of the moveable element 34 such that the orientation corresponds to the actual driving direction of the vehicle such to match visual information and vestibular information of any vehicle passenger.

The control unit 32 provides control information to the actuator 33, which actually controls the moveable element 34. For example, the actuator 34 may be an electrical motor driving a cover similar to the cover 22 as illustrated in Figs. 3A and 3B. However, the term 'actuator' may be interpreted broadly as an element configured and arranged to affect an orientation of a visible object.

As apparent to those skilled in the art, the device according to the present invention may be combined with or incorporated in any other device or unit in the vehicle. For example, a sensor may be provided in the device for monitoring an interior condition, e.g. presence of a passenger. Further, the moveable and visible element may take any other form or shape that provides suitable visual information to a passenger.

Detailed embodiments of the present invention are disclosed herein; however, it is to be understood that the disclosed embodiments are merely exemplary of the invention, which can be embodied in various forms. Therefore, specific structural and functional details disclosed herein are not to be interpreted as limiting, but merely as a basis for the claims and as a representative basis for teaching one skilled in the art to variously employ the present invention in expectedly any appropriately detailed structure. In particular, features presented and described in separate dependent claims may be applied in combination and any advantageous combination of such claims are herewith disclosed.

Further, it is contemplated that structural elements may be generated by application of three-dimensional (3D) printing techniques. Therefore, any reference to a structural element is intended to encompass any computer executable instructions that instruct a computer to generate such a structural element by three-dimensional printing techniques or similar computer controlled manufacturing techniques. Furthermore, any such reference to a structural element is also intended to encompass a computer readable medium carrying such computer executable instructions.

Further, the terms and phrases used herein are not intended to be limiting, but rather to provide an understandable description of the invention. The terms "a" or "an", as used herein, are defined as one or more than one. The term plurality, as used herein, is defined as two or more than two. The term another, as used herein, is defined as at least a second or more. The terms including and/or having, as used herein, are defined as comprising (i.e., open language). The term coupled, as used herein, is defined as connected, although not necessarily directly.

## Claims

1. A roof assembly for a vehicle, wherein the roof assembly comprises a support frame (5) mountable on a body frame of the vehicle to form a roof of a passenger compartment of the vehicle, the roof assembly comprising a device (20, 30) configured to be arranged in a passenger compartment of a vehicle, wherein at least a part (22, 34) of the device (20, 30) is configured to be moveable in a predetermined limited range of movement corresponding to an acceleration of the vehicle and wherein the device (20, 30) is designed and configured to provide visual information to a passenger with respect to a direction of such acceleration by movement of said part of the device (22, 34) corresponding to such direction, **characterised in that** the device comprises a support element (24), an elastic element (26a, 26b), and said moveable part (22), wherein the support element (24) is fixedly mounted in the passenger compartment and wherein the elastic element (26a, 26b) is arranged between the support element (24) and the moveable part (22), the moveable part (22) being configured to move in response to acceleration induced inertia of the moveable part (22) and the elastic element (26a, 26b) is arranged to counteract a relative movement between the moveable part (22) and the support element (24).

2. The assembly according to claim 1, wherein said part of the device (20) is a decorative element.

3. The assembly according to claim 1, wherein said part of the device (22, 34) is arranged on a roof of the passenger compartment.

4. The assembly according to claim 1, wherein said part of the device (22, 34) is rotatably mounted in a substantially horizontal plane such to rotate with a rotational movement of the vehicle in the substantially horizontal plane.

5. The assembly according to claim 1, wherein said part of the device (22, 34) is rotatably mounted in a substantially vertical plane such to rotate with a rotational movement of the vehicle in the substantially vertical plane.

6. The assembly according to claim 1, wherein the device comprises a control unit, wherein the control unit comprises a sensor for detecting the acceleration and an actuator for moving said part of the device corresponding to the acceleration as detected.

7. The assembly according to claim 1, wherein the device comprises a sensor for detecting a condition in the passenger compartment.

8. The assembly according to claim 1, wherein said moveable part (22) is designed such that a centre of gravity of the moveable part (22) is aligned with a centre of rotation in a main moving direction of the vehicle.

9. A computer program product comprising instructions, which when the program is executed by a processing unit of the control unit according to claim 6, cause said processing unit to execute the step of detecting an acceleration and the step of moving said part of the device corresponding to the acceleration as detected.

10. A computer readable medium having stored there on the computer program product according to claim 9.

## Patentansprüche

1. Dach-Einrichtung für ein Fahrzeug, wobei die Dach-Einrichtung aufweist einen Halterahmen (5), der an einem Karosserierahmen des Fahrzeugs montierbar ist, um ein Dach eines Fahrgastraums des Fahrzeugs zu bilden, wobei die Dach-Einrichtung aufweist eine Vorrichtung (20, 30), die eingerichtet ist, um in einem Fahrgastraum eines Fahrzeugs angeordnet zu sein, wobei wenigstens ein Teil (22, 34) der Vorrichtung (20, 30) eingerichtet ist, um in einem vorbestimmten begrenzten Bewegungsbereich bewegbar zu sein korrespondierend zu einer Beschleunigung des Fahrzeugs, und wobei die Vorrichtung (20, 30) gestaltet und eingerichtet ist, um einem Fahrgast visuelle Informationen bezüglich einer Richtung einer solchen Beschleunigung bereitzustellen durch Bewegung dieses Teils der Vorrichtung (22, 34) korrespondierend zu einer solchen Richtung, **dadurch gekennzeichnet, dass** die Vorrichtung aufweist ein Halteelement (24), ein elastisches Element (26a, 26b) und den bewegbaren Teil (22), wobei das Haltelement (24) im Fahrgastraum fest montiert ist und wobei das elastische Element (26a, 26b) zwischen dem Halteelement (24) und dem bewegbaren Teil (22) angeordnet ist, wobei der bewegbare Teil (22) eingerichtet ist, um sich in Antwort auf Beschleunigung-induzierte Trägheit des bewegbaren Teils (22) zu bewegen, und das elastische Element (26a, 26b) angeordnet ist, um einer Relativbewegung zwischen dem bewegbaren Teil (22) und dem Halteelement (24) entgegenzuwirken.

2. Einrichtung gemäß Anspruch 1, wobei der Teil der Vorrichtung (20) ein dekoratives Element ist.

3. Einrichtung gemäß Anspruch 1, wobei der Teil der Vorrichtung (22, 34) an einem Dach des Fahrgastraums angeordnet ist.

4. Einrichtung gemäß Anspruch 1, wobei der Teil der Vorrichtung (22, 34) in einer im Wesentlichen horizontalen Ebene rotierbar montiert ist, um bei einer Rotationsbewegung des Fahrzeugs in der im Wesentlichen horizontalen Ebene zu rotieren.

5. Einrichtung gemäß Anspruch 1, wobei der Teil der Vorrichtung (22, 34) in einer im Wesentlichen vertikalen Ebene rotierbar montiert ist, um bei einer Rotationsbewegung des Fahrzeugs in der im Wesentlichen vertikalen Ebene zu rotieren.

6. Einrichtung gemäß Anspruch 1, wobei die Vorrichtung eine Steuereinheit aufweist, wobei die Steuereinheit aufweist einen Sensor zum Detektieren der Beschleunigung und einen Aktuator zum Bewegen des Teils der Vorrichtung korrespondierend zu der Beschleunigung wie detektiert.

7. Einrichtung gemäß Anspruch 1, wobei die Vorrichtung aufweist einen Sensor zum Detektieren eines Zustands im Fahrgastraum.

8. Einrichtung gemäß Anspruch 1, wobei der bewegbare Teil (22) derart gestaltet ist, dass ein Schwerpunkt des bewegbaren Teils (22) mit einem Rotationsmittelpunkt in einer Hauptbewegungsrichtung des Fahrzeugs ausgerichtet ist.

9. Computerprogrammprodukt, das Instruktionen aufweist, die, wenn das Programm von einer Prozesseinheit der Steuereinheit gemäß Anspruch 6 ausgeführt wird, die Prozesseinheit veranlassen, den Schritt des Detektierens einer Beschleunigung und den Schritt des Bewegens des Teils der Vorrichtung korrespondierend zu der Beschleunigung wie detektiert auszuführen.

10. Computerlesbares Medium, welches das Computerprogrammprodukt gemäß Anspruch 9 darauf gespeichert hat.

## Revendications

1. Ensemble toit pour un véhicule, dans lequel l'ensemble toit comprend un cadre de support (5) pouvant être monté sur un châssis de carrosserie du véhicule pour former un toit d'un habitacle du véhicule, l'ensemble toit comprenant un dispositif (20, 30) configuré pour être agencé dans un habitacle d'un véhicule, dans lequel au moins une partie (22, 34) du dispositif (20, 30) est configurée pour être mobile dans une amplitude de mouvement limitée prédéterminée correspondant à une accélération du véhicule et dans lequel le véhicule (20, 30) est conçu et configuré pour fournir des informations visuelles à un passager par rapport à une direction d'une telle accélération par déplacement de ladite partie du dispositif (22, 34) correspondant à une telle direction, **caractérisé en ce que** le dispositif comprend un élément de support (24), un élément élastique (26a, 26b) et ladite partie mobile (22), dans lequel l'élément de support (24) est monté de manière fixe dans l'habitacle et dans lequel l'élément élastique (26a, 26b) est agencé entre l'élément de support (24) et la partie mobile (22), la partie mobile (22) étant configurée pour se déplacer en réponse à l'inertie induite par l'accélération de la partie mobile (22) et l'élément élastique (26a, 26b) est agencé pour compenser un déplacement relatif entre la partie mobile (22) et l'élément de support (24).

2. Ensemble selon la revendication 1, dans lequel ladite partie du dispositif (20) est un élément décoratif.

3. Ensemble selon la revendication 1, dans lequel ladite partie du dispositif (22, 34) est agencée sur un toit de l'habitacle.

4. Ensemble selon la revendication 1, dans lequel ladite partie du dispositif (22, 34) est montée de manière rotative dans un plan sensiblement horizontal de façon à tourner avec un mouvement de rotation du véhicule dans le plan sensiblement horizontal.

5. Ensemble selon la revendication 1, dans lequel ladite partie du dispositif (22, 34) est montée de manière rotative dans un plan sensiblement vertical de façon à tourner avec un mouvement de rotation du véhicule dans le plan sensiblement vertical.

6. Ensemble selon la revendication 1, dans lequel le dispositif comprend une unité de commande, dans lequel l'unité de commande comprend un capteur pour détecter l'accélération et un actionneur pour déplacer ladite partie du dispositif correspondant à l'accélération telle que détectée.

7. Ensemble selon la revendication 1, dans lequel le dispositif comprend un capteur pour détecter une condition dans l'habitacle.

8. Ensemble selon la revendication 1, dans lequel ladite partie mobile (22) est conçue de sorte qu'un centre de gravité de la partie mobile (22) soit aligné sur un centre de rotation dans une direction de déplacement principale du véhicule.

9. Produit de programme informatique comprenant des instructions qui, lorsque le programme est exécuté par une unité de traitement de l'unité de commande selon la revendication 6, amènent ladite unité de traitement à exécuter l'étape de détection d'une accélération et l'étape de déplacement de ladite partie du dispositif correspondant à l'accélération telle que détectée.

10. Support lisible par ordinateur sur lequel est stocké le produit de programme informatique selon la revendication 9.
